# EUROPEAN PATENT APPLICATION

(11) **EP 4 000 600 A1**
(43) Date of publication of application: **25.05.2022**
(21) Application number: 20383022.9
(22) Date of filing: 24.11.2020
(51) Int. Cl.: A61K 9/06, A61K 9/08, A61K 31/4709, A61K 41/00

(54) **COMBINED THERAPY AGAINST CANCER**

(71) Applicant: Universitat Politècnica de Catalunya, 08034 Barcelona (ES)
(72) Inventor: CANAL BARNILS, Cristina, 08034 Barcelona (ES); TORNÍN CAVIELLES, Juan, 33520 Nava (ES)
(74) Representative: Elzaburu S.L.P.

(57) **Abstract**

The present invention relates to a composition comprising a liquid or a hydrogel comprising reactive oxygen and nitrogen species (RONS) and a STAT3 inhibitor, as well as to the use of said composition in the treatment of cancer.

## Description

### Field of the invention

The present invention belongs to the field of Biotechnology and relates to a combined therapy against cancer.

### Background of the invention

Liquids treated with cold atmospheric plasma (CAP) have been used in anti-cancer therapy. Interestingly, CAP has shown to selectively kill cancer cells, without affecting healthy cells. It has been described that the cytotoxicity of the CAP treated liquid depends on the amount of reactive oxygen and nitrogen species (RONS) in it (Bauer, G. et al. Sci Rep 9, 14210 (2019); Tornin, J. et al. Sci Rep 9, 10681, (2019)).

STAT-3 inhibitors have also been assayed in anti-cancer therapy. It has been disclosed the high incidence of STAT3 tyrosine phosphorylation in osteosarcoma cell lines and that targeting STAT3 using STAT3 inhibitor S3I-201 in osteosarcoma cell lines showed significant inhibition of cell growth and colony formation, as well as apoptosis enhancement via the caspase-3 pathway *in vitro* (Wang et al. Anticancer Research 34: 6537-6546 (2014)).

In anti-cancer therapy, there is still a need to find new treatments that kill cancer cells without affecting healthy cells. CAP has been used and has proven to be effective in different cancer types, including osteosarcoma (OS). OS is the most common pediatric bone primary tumor in the world and the eighth most common childhood cancer. OS is a very aggressive tumor and shows high capacity to metastasize, however current therapies have not advanced much in the last 30 years. Current treatments include a first surgery combined with high doses of methotrexate, cisplatin, doxorubicin or Ifosfamide. Due to the difficult access to surgery resection and the harmful effects of chemotherapy for OS, there is an urgent need to evaluate new treatments that improve both cure and survival in this disease.

### Description of the invention

The present invention provides a solution to the above-mentioned problem. The inventors have surprisingly found that the combination of oxidative stress-based therapies like cold plasma treated liquids or hydrogels with a STAT3 inhibitor is synergistically effective as cancer therapy, dramatically preventing the growth of cancer cells, both cancer stem cells and non-cancer stem cells, while not affecting healthy cells. This allows the use of non-toxic concentrations of both comprising reactive oxygen and nitrogen species (RONS) and a STAT3 inhibitor to achieve a high cytotoxic effect only on cancer cells.

In a first aspect, the present invention relates to a composition comprising:
a. a liquid or a hydrogel comprising RONS; and
b. a STAT3 inhibitor.

In a preferred embodiment, the RONS comprise between 10 and 3000 µM H₂O₂, preferably between 10 and 600 µM H₂O₂, more preferably between 10 and 300 µM H₂O₂, even more preferably between 20 and 250 µM H₂O₂ In another preferred embodiment, the RONS comprise between 10 and 800 µM NO₂⁻, preferably between 10 and 400 µM NO₂⁻, more preferably between 10 and 250 µM NO₂⁻, even more preferably between 20 and 250 µM NO₂⁻. In a preferred embodiment, the RONS comprise between 10 and 3000 µM H₂O₂ and/or between 10 and 800 µM NO₂⁻. In another preferred embodiment, the RONS comprise between 10 and 600 µM H₂O₂ and/or between 10 and 400 µM NO₂⁻. In another preferred embodiment, the RONS comprise between 10 and 300 µM H₂O₂ and/or between 10 and 250 µM NO₂⁻. In another preferred embodiment, the RONS comprise between 20 and 250 µM H₂O₂ and/or between 20 and 250 µM NO₂⁻.

The RONS concentration is quantified either using the AR/HRP reagent method or the Griess reagent method for H₂O₂ and NO₂⁻, respectively. Also, plastic strips with test paper which allow quantification of H₂O₂ based on a redox reaction and NO₂⁻, also using the Griess reagent may be used when a hydrogel is used and the protein solution causes interferences with the AR/HRP reagent method or the Griess reagent method. These two methods give equivalent results.

In a preferred embodiment, the composition comprises a liquid comprising RONS, wherein the liquid is an aqueous medium. The aqueous medium may be selected from water, saline aqueous solutions such as Ringer's solution, parenteral solution for hospital use, solutions used as drug vehicles or cell culture media.

In a preferred embodiment, the composition comprises an hydrogel comprising RONS, wherein the hydrogel is an aqueous solution comprising at least one of gelatin, a gelatin derivative such as metacrylated gelatin, fibrin, fibronectin, collagen, a collagen derivative, alginate, agarose, cellulose, modified cellulose such as hydroxypropyl cellulose, carboxymethylcellulose or hydroxyethyl cellulose, xantan gum, polyethyleneglycol, hyaluronic acid, chitosan, polylactide-co-glycolide, polyhydroxyalcanoates. In a preferred embodiment, the hydrogel is an aqueous solution comprising gelatin, alginate, collagen or mixtures thereof.

In a preferred embodiment, the composition further comprises a ceramic material comprising calcium. Preferably, the ceramic material comprising calcium is selected from calcium phosphate, hydroxyapatite, calcium deficient hydroxyapatite, brushite, fluorapatite, calcium- sodium and potassium- phosphate, calcium- and sodium- phosphate, calcium- and potassium- phosphate, calcium pyrophosphate, calcium carbonate, calcium sulphate, calcium sulphate hemihydrate, calcium oxide, calcium hydroxide, and mixtures thereof, preferably the ceramic material is hydroxyapatite, brushite, tricalcium phosphate or mixtures thereof.

In a preferred embodiment, the STAT3 inhibitor prevents Stat3 expression, STAT3 phosphorylation, STAT3 dimerization, STAT3 translocation to the nucleus, STAT3 DNA binding or STAT3 mediated transcription. In a preferred embodiment, the STAT3 inhibitor prevents STAT3 phosphorylation, more preferably the STAT3 inhibitor prevents STAT3 phosphorylation at tyrosine 705. The STAT3 inhibitor can be selected from S3I-201, WP1066, Resveratrol, Stattic, Niclosamide, STAT3-IN-1, STAT5-IN-1, AS1517499, C188-9, BP-1-102, SH-4-54, Cryptotanshinone, Bosutinib (SKI-606), Fludarabine, Nifuroxazide, Brevilin A, RCM-1, Kaempferol-3-O-rutinoside, Cucurbitacin IIb, SC-43, Scutellarin, HJC0152, SH5-07 (SH-5-07), APTSTAT3-9R, Ochromycinone (STA-21), Napabucasin (BBI608), HO-3867, Artesunate or any combination thereof. In a preferred embodiment, the STAT3 inhibitor is S3I-201 or BBI608. In a preferred embodiment, the STAT3 inhibitor is S3I-201. In another preferred embodiment, the STAT3 inhibitor is BBI608.

In a preferred embodiment, the composition of the invention further comprises at least an active agent selected from a chemotherapeutic agent and an immunotherapeutic agent.

A second aspect of the present invention relates to the composition of the first aspect for use in the treatment of cancer. In a preferred embodiment, the cancer is selected from bone cancer, sarcoma, prostate cancer, urotelioma, breast cancer, brain cancer, or colon cancer. In a more preferred embodiment, the bone cancer is osteosarcoma.

In a preferred embodiment of the second aspect, the liquid or hydrogel comprising RONS and the STAT3 inhibitor are administered simultaneously or sequentially. In another preferred embodiment, the composition is administered before or after surgery.

In another aspect, the present invention relates to a method of treating cancerous tissue in a subject comprising administering to the subject a composition comprising:
(a) a liquid or a hydrogel and reactive oxygen and nitrogen species (RONS); and
(b) a STAT3 inhibitor.

In a preferred embodiment, components (a) and (b) are administered either simultaneously or subsequently.

In another aspect, the present invention relates to a method of treating cancerous tissue in a subject comprising applying cold atmospheric plasma to said tissue and administering a STAT3 inhibitor to said subject. The cancerous tissue is preferably osteosarcoma.

### Brief description of the drawings

**Figure 1****.** Concentration of RONS (µM) in plasma treated liquid, where squares indicate micromolar (µM) concentration of Hydrogen Peroxide (H₂O₂) by AmplexRed/HRP assay and columns indicate the micromolar (µM) concentration of Nitrites (NO₂⁻) by Griess assay. Data are presented as mean, n=3.

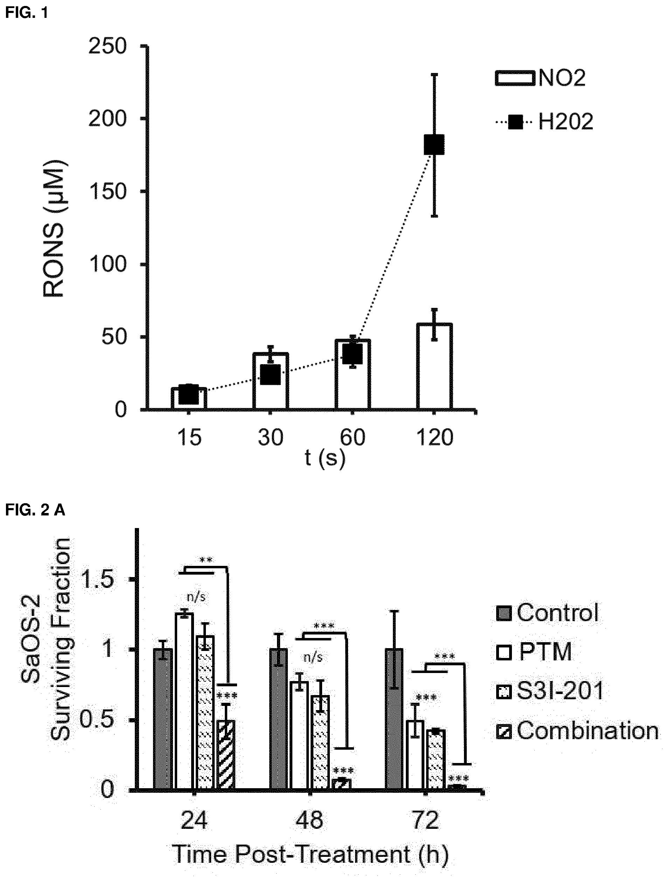

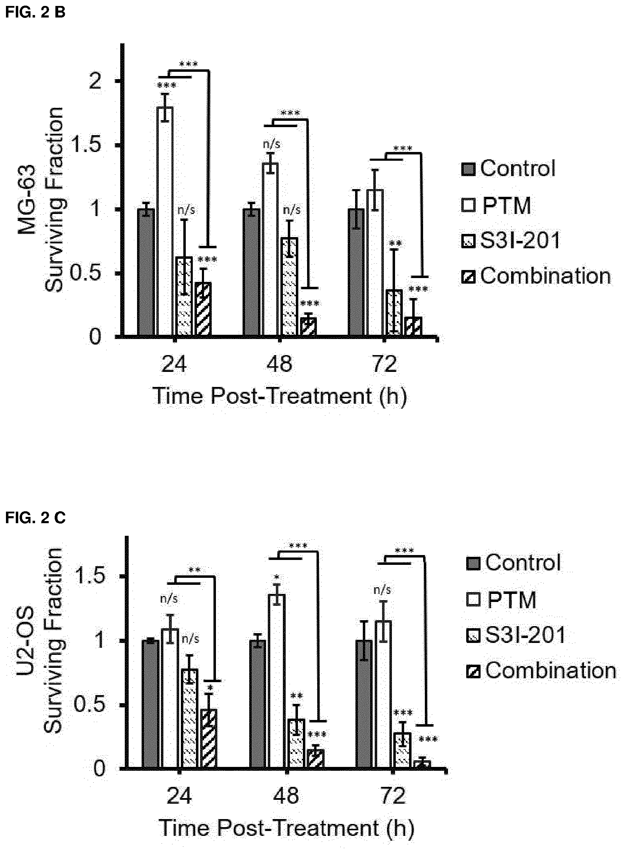

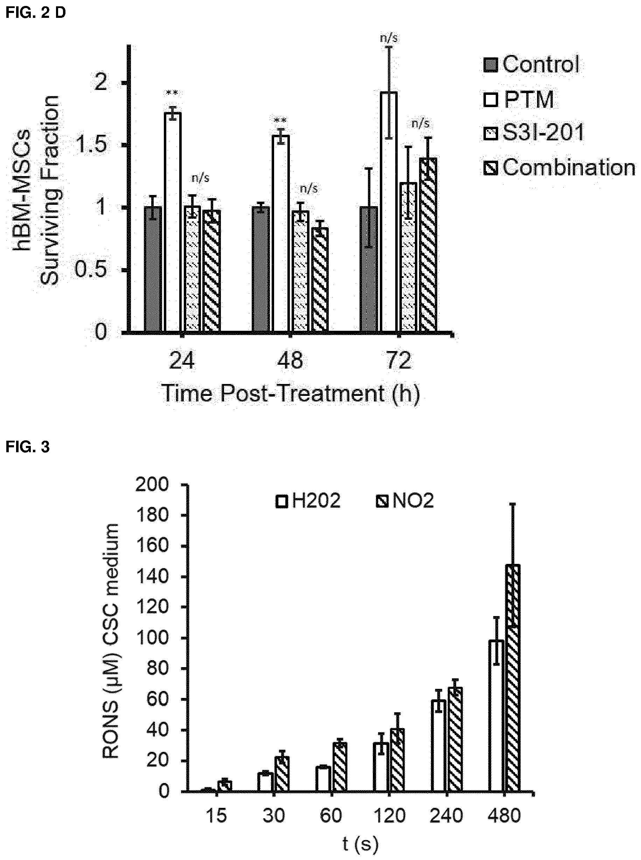

**Figure 2****.** Synergic effect of the combined therapy on cell viability. Cell viability (WST1 assay) measured after the treatment of the indicated cell lines (A. SaOS-2. B. MG-63. C. U2-OS. D. hBM-MSCs) using 15s - plasma treated medium (PTM), S3I-201 (80 µM) or a combination of both for 24, 48 and 72 hours. For each time, the four columns from left to right correspond to control, plasma treated medium (PTM), S3I-201 and combination of plasma treated medium and S3I-201. Cell viability is expressed relative to the corresponding control. Data represent the mean and standard deviation of n=3 independent experiments.
**Figure 3****.** Concentration of RONS in CSC culture. Columns indicate micromolar (µM) concentration of Hydrogen Peroxide (H₂O₂) by AmplexRed/HRP assay and markers the micromolar (µM) concentration of Nitrites (NO₂⁻) by Griess assay on indicated treatment times. Data are presented as mean, n=3.
**Figure 4****.** STAT3 inhibitor and plasma treated medium act synergistically preventing the growth of osteosarcoma cell line derived tumorspheres. Tumorsphere number from MG-63 cells. n=3 independent replicates, and each replicate from n=6 independent cultures.

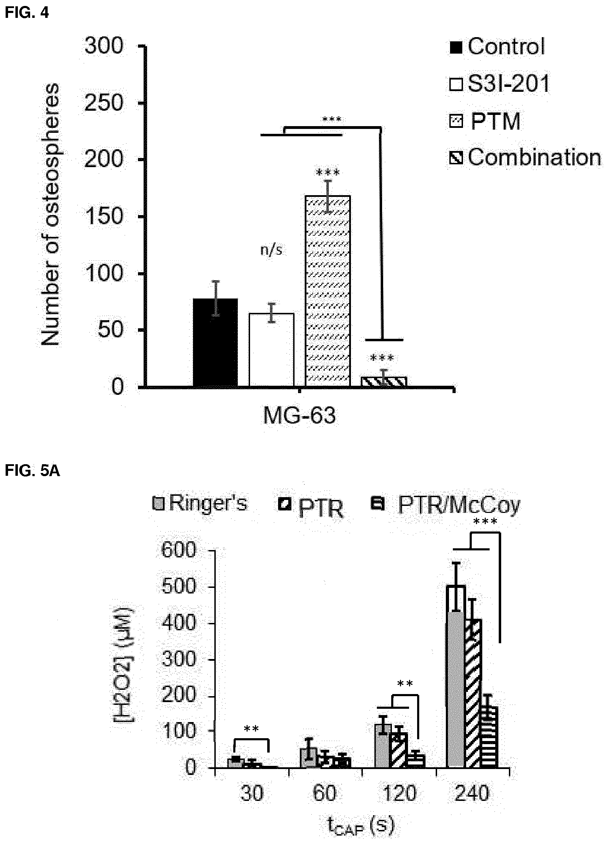

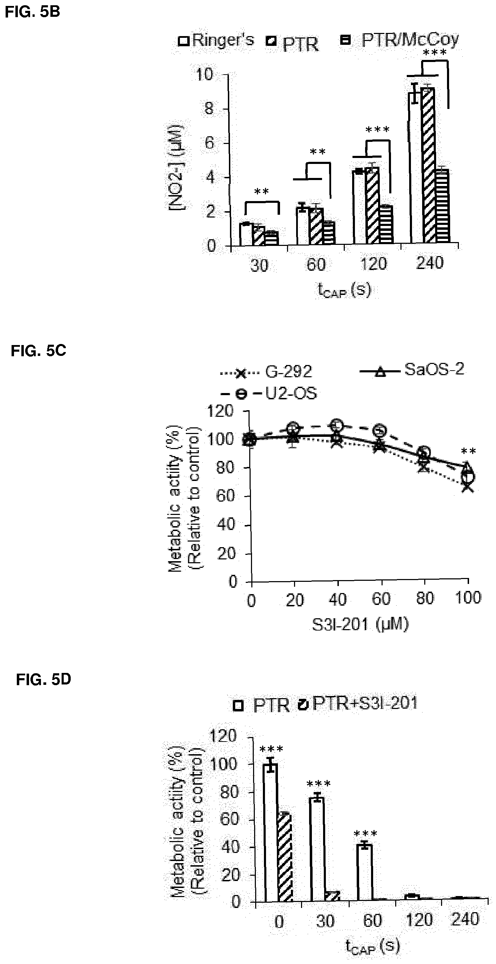

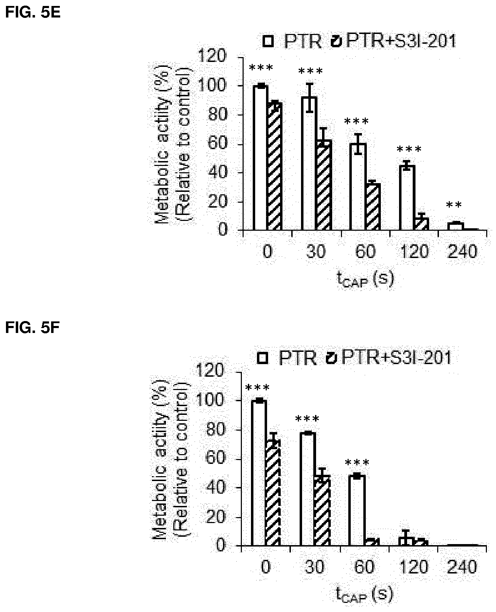

**Figure 5****.** Synergistic effect of PAR with S3I-201 reducing cell viability in OS cell lines in adherent culture. Concentration of H₂O₂ (A) and NO₂⁻ (B) measured in plasma-treated Ringer's saline (PTR) before and after the addition of 10% of FBS and after diluting 1:1 in McCoy's A5 Medium Modified. G-292, SaOS-2 and U2-OS cells in adherent culture were exposed during 2 hours (C) to different concentrations of S3I-201 (20-100 µM) in untreated PTR and to (D-F) PTR treated for 30-240 seconds with and without the addition of 100 µM of S3I-201 after treatment. After that, PTR was diluted 1:1 in McCoy's A5 Medium Modified. Metabolic activity was determined 72 hours after PTR exposure by PrestoBlue assay. Values were relativized to cells exposed to untreated PTR.

### Examples

The following examples are provided to further illustrate, but not to limit this invention.

### Plasma treatment and RONS concentration

The generation of RONS in the cell culture medium following plasma treatment is time dependent and shows an equilibrated quantity of NO₂⁻ and H₂O₂ (Figure 1). Most treatment times do not lead to significant differences on the concentration of NO₂⁻ except for 120s where the concentration of H₂O₂ generated in plasma treated medium is up to 3 times higher than NO₂⁻ (Figure 1). These concentrations are the ones used in the next example, where 15s-plasma treated medium was used.

### S3I-201 and plasma treated medium act synergistically preventing the growth of osteosarcoma cell lines but do not affect healthy cells

The viability of osteosarcoma SaOS-2, MG-63 and U2-OS cells and healthy hBM-MSCs is shown in Figure 2. 15s-plasma treated medium was cytotoxic only to SaOS-2 cells whereas MG-63, U2-OS and hBM-MSCs cells showed an increase in cell proliferation (Figures 2 A-D). Surprisingly, the combination of 15s-plasma treated medium and the STAT3 inhibitor S3I-201 significantly showed a synergistically cytotoxic effect than separate treatments. The combined treatment increased the cytotoxicity of plasma treated medium in the three osteosarcoma cell lines even at very low dose of plasma treated medium-15s, while healthy cells were not affected (Figures 2 A-D).

### S3I-201 and plasma treated medium prevents growth of cancer stem cell (CSC) cultures.

The cytotoxic potential of a plasma treated medium (DMEM-F12) was tested over 3D monoclonal osteospheres. The generation of RONS in the cell culture medium following plasma treatment is time dependent and shows an equilibrated cocktail of NO₂⁻ and H₂O₂ in all treatment times investigated (Figure 3).

We directly treated already formed osteospheres of MG-63 cells at day 7 post-seeding with a combination of 480s-plasma treated medium with STAT3 inhibitor S3I-201 for 3 days. Notably, the combination of S3I-201 and 480s-plasma treated medium, was completely effective reducing the number of osteospheres (Figure 4).

### Synergistic effect of plasma treated medium with S3I-201 reducing cell viability in OS cell lines in adherent culture.

Concentration of H₂O₂ (Figure 5A) and NO₂⁻ (Figure 5B) were measured in plasma-treated Ringer's saline (PTR) before and after the addition of 10% of FBS and after diluting 1:1 in McCoy's A5 Medium Modified. G-292, SaOS-2 and U2-OS cells in adherent culture were exposed during 2 hours to (Figure 5C) different concentrations of S3I-201 (20-100 µM) in untreated PTR and to (Figures 5D-F) PTR treated for 30-240 seconds with and without the addition of 100 µM of S3I-201 after treatment. After that, PTR was diluted 1:1 in McCoy's A5 Medium Modified. Metabolic activity was determined 72 hours after PTR exposure by PrestoBlue assay. Values were relativized to cells exposed to untreated PTR.

### Cytotoxic effect of plasma treated hydrogel solutions

A 50/50 blend of 0.5 weight % alginate and 2 weight % gelatin solutions were prepared (final concentration of 0.25 % wt alginate and 1 % wt gelatin). The mixture of alginate/gelatin was prepared is by vortexing in a ratio 1:1, 2 % wt gelatin with 0.5 % wt alginate for 2 minutes. Gelatin in powder is mixed with MilliQ water at 37°C using magnetic stirring for 2 hours to obtain a 2 % wt gelatin gel. 0.5% alginate was prepared by mixing alginate powder with MilliQ water using a SpeedMixer^{™} DAC 150.1 FVZ-K (SpeedMixer^{™}, Germany) at 3500 r.p.m. for 15 min. The 0.25 % wt alginate and 1 % wt gelatin aqueous mixture was treated with an atmospheric pressure plasma jet kINPen IND^{®} (Neoplas, Germany) operating with Argon to generate plasma. Treatment conditions: 1 L/min gas flow, 10 mm nozzle distance, and 180 seconds treatment. Treatment performed in 200 µL of mixture in a 96-well plate. Said plasma-treated mixture produced the following concentrations of reactive species in the material:

| | **H₂O₂ (mg/L)** | **NO₂⁻ (mg/L)** | **NO₃⁻ (mg/L)** |
|---|---|---|---|
| **Water** | 10.3 | 2.6 | - |
| **Hydrogel solution** | 16.7 | 17.0 | 124.0 |

As shown in the table, the values of reactive species obtained in this composition are several-fold higher than those generated in water. Said plasma-treated mixture was used in cell viability assays in both an osteosarcoma cell line (SaOS-2) and in healthy cells (human bone marrow mesenchymal stem cells or hBM-MSC):

| **Hydrogel solution** | **Cell viability at 72 h (%)** |
|---|---|
| **SaOS-2** | 40.94 ± 3.44 |
| **hBM-MSC** | 90.57 ± 8.19 |

This composition shows selectivity of the plasma-treated polymer solution on the cancer cell line, allowing the survival of healthy cells (hBM-MSC) after 72 hours.

### Cytotoxic effect of plasma treated hydrogel solutions with ceramic material

The composition of the preceding example was prepared by treating with plasma during 5 minutes instead of 3 minutes, and further comprising 5 % wt of calcium deficient hydroxyapatite microspheres (MS), which were added and mixed in the vortex for 2 min. The diameter of the microspheres was 100 µm<Ø<150 µm. The amount of RONS was not affected by the addition of the bioceramic material. The concentration of reactive species generated by plasma in the polymer solution and in the composition after adding the bioceramic material is equivalent, as can be seen below:

| **Example** | **[H₂O₂] (mg/L)** | **[NO₂⁻] (mg/L)** | **[NO₃⁻] (mg/L)** |
|---|---|---|---|
| **Hydrogel solution** | 78.0 ± 15.6 | 20.0 ± 4.0 | 297.0 ± 59.4 |
| **Hydrogel solution + 5% microspheres** | 84.7 ± 16.9 | 21.5 ± 4.3 | 270.0 ± 54.0 |

The species generated in the composition can be released to a surrounding media and preserved at least for 24 hours. This was also tested with a composition where the MS were previously loaded with the active agent doxorubicin (DOX):

| | **H₂O₂ concentration in 1 mL release media (mg/L)** | | |
|---|---|---|---|
| **Time (h)** | **Hydrogel** | **Hydrogel+MS** | **Hydrogel with DOX-loaded MS** |
| **0** | 0 | 0 | 0 |
| **0.5** | 2.37 ± 0.15 | 3.11 ± 0.19 | 3.18 ± 0.11 |
| **1** | 2.55 ± 0.37 | 4.18 ± 0.39 | 2.57 ± 0.09 |
| **2** | 1.99 ± 0.34 | 3.50 ± 0.25 | 2.64 ± 0.09 |
| **4** | 2.08 ± 0.33 | 3.76 ± 0.39 | 3.06 ± 0.10 |
| **24** | 1.95 ± 0.23 | 3.06 ± 0.64 | 2.21 ± 0.08 |

| | **NO₂⁻ concentration in 1 mL release media (mg/L)** | | |
|---|---|---|---|
| **Time (h)** | **Hydrogel** | **Hydrogel+MS** | **Hydrogel with DOX-loaded MS** |
| **0** | 0 | 0 | 0 |
| **0.5** | 0.25 ± 0.02 | 0.25 ± 0.03 | 0.25 ± 0.05 |
| **1** | 0.31 ± 0.02 | 0.36 ± 0.04 | 0.29 ± 0.06 |
| **2** | 0.38 ± 0.01 | 0.46 ± 0.01 | 0.35 ± 0.07 |
| **4** | 0.43 ± 0.02 | 0.51 ± 0.03 | 0.29 ± 0.06 |
| **24** | 0.54 ± 0.06 | 0.60 ± 0.04 | 0.32 ± 0.06 |

Said Hydrogel + MS was used in cell viability assays in osteosarcoma cell line (SaOS-2):

| | **SaOS-2 cell viability at 24 h** (%) | **SaOS-2 cell viability at 72 h** (%) |
|---|---|---|
| **Untreated composition** | 93.6 ± 6.8 | 96.7 ± 2.1 |
| **Hydrogel + MS** | 13.8 ± 1.3 | 7.5 ± 5.5 |

### Materials and Methods

### Cell culture and drugs

We evaluated the effects of cold plasma treated medium on the osteosarcoma cell lines SaOS-2, MG-63 and U2-OS vs healthy hBM-MSCs (both cell types obtained from ATCC, USA). In this study cell lines were grown in Dulbecco's Modified Eagle Medium (DMEM) with glucose (4,5g/L), pyruvate, no glutamine (Gibco^{™}, USA), with 10% fetal bovine serum (FBS) (Gibco^{™} cat no. 10270098, USA), 2mM L-glutamine (Gibco^{™}), 100 units/mL penicillin (Gibco^{™}) and 100 µg/mL streptomycin (Gibco^{™}). The cells were incubated at 37 °C, 95% humidity and 5% CO₂. Also, G-292, SaOS-2 and U2-OS cells were cultured in McCoy's A5 Medium Modified with 1,5 mM L-glutamine (GibcoTM, Carlsbad, CA, USA) supplemented with 10% of fetal bovine serum (FBS), penicillin/streptomycin (50 U/mL and 50 µg/mL, respectively) and 1 mM sodium pyruvate, all from GibcoTM. S3I-201 (cat. No S1155) was obtained from Selleckhem.

### Cold-plasma jet device and application to culture medium or monolayer culture.

kINPen^{®} IND (Neoplas tools GmbH, Greifswald, Germany) is a commercial plasma jet used in clinics that consists of a hand-held unit that discharges plasma under atmospheric conditions, employing a DC power unit and Argon gas to generate the plasma. In the centre of a ceramic capillary (inner diameter 1.6 mm) a pin-type electrode (1 mm diameter) is mounted, and a ring around the dielectric as grounded counter-electrode. The needle is powered by a small RF generator producing a sinusoidal voltage waveform ranging from 2 kV to 3 kV amplitude peak at a frequency of 1 MHz and modulated with 2.5 kHz and a plasma duty cycle of 1:1.

To apply the plasma directly or to treat the medium with the plasma, we designed a protocol that allowed us compare between methods under the same operation parameters: Argon flow of 3 L/min, at a distance of 10 mm between the surface of the liquid and the jet nozzle, during 15,30,60, 120,240 or 480 seconds over 1mL of culture medium (for example, DMEM, high glucose, no glutamine, no phenol red without 0.1g/L Sodium Pyruvate (GibcoTM , cat.no 11360070, Carlsbad, CA, USA), or DMEM-F12) in a 24-well plate containing 30.000 cells/well.

To produce the plasma-treated Ringer's saline (PTR), 2 mL of sterile Ringer's saline (8.6 g/L NaCl, 0.33 g/L CaCl₂ and 0.3 g/L KCl) were placed under the plasma jet at room temperature with a gas flow of 3 L/min and a distance of 10 mm from the jet nozzle in sterile conditions. The liquid was placed in multiple well plates of 1.9 cm² of surface (24 well-plates). Plasma treatment times between 30-240 seconds were investigated. A 10% of FBS was added immediately after treatment.

### Metabolic Activity

Subconfluent G-292, SaOS-2 and U2-OS cells were trypsinized, centrifuged and seeded in 48-well plates at a density of 15x 10³ cells/well, and incubated in 300 µL of their corresponding medium for 24 h. On the one hand, the culture medium was replaced in each cell line after incubation by 300 µL of Ringer's with 10% of FBS with different concentrations (20-100 µM) of S3I-201. On the other hand, the culture medium was replaced in each cell line by 300 µL of PTR treated during 30-240 seconds with and without S3I-201 (100 µM). Cells were incubated during two hours for each condition in triplicate. As a positive control, each cell line was incubated during two hours with Ringer's supplemented with 10% of FBS. Afterwards, 300 µL of corresponding fresh medium was added in each condition. Cells were then incubated at 37 °C for 72 hours. Cell metabolism was evaluated by PrestoBlue assay; 20% of PrestoBlue reagent in culture media were employed. As a negative control, PrestoBlue was incubated without cells. Fluorescence were measured with λex/em of 530/590 nm and fluorescence from negative control was subtracted. Fluorescence of each treated condition was referenced to positive control.

### Determination of RONS

We determined the concentration of Hydrogen Peroxide, and Nitrites in plasma treated culture media, following the same protocol described before in Tornin, J. et al. Sci Rep 9, 10681, (2019). Briefly, nitrite concentration was performed using Griess reagent and the concentration of hydrogen peroxide was determined by a redox reaction using a coloured reagent and Horseradish Peroxidase.

### Monolayer cell viability assay and immunofluorescence

To evaluate the antitumor effects, a WST-1 (Roche, Germany) cell proliferation assay was performed according to the manufacturer's instructions. Cells were seeded in a 24-well plate at a density of 30×10³ cells per 1000µL of culture medium. On the following day, the culture medium was replaced with 1000µL of plasma treated medium. After 24.48 or 72 hours, WST-1 working solution (18µL/mL) was added to each well and plates were incubated at 37°C for 60 min. Absorbance was measured at λ_{abs} =440 nm. Each experiment was performed by independent triplicates. Medium untreated with cold plasma was used as control.

### Culture of osteospheres and viability

MG-63 cells line was plated at a density of 1.500 cells per well in UltraLow Costar 6-well plates (Corning) to prevent cell attachment, in serum-free sphere medium containing DMEM-F12+Glutamax (Gibco), B-27/VitA Supplement (1:50; Life Technologies), Heparin (1:1000; Sigma), the growth factors human EGF (20 ng/ml) and human bFGF (10 ng/ml; GoldBio) and 1 % methylcellulose (Sigma) to avoid cell aggregation. In addition, fresh aliquots of EGF and bFGF were added every three days. To analyze the effects of plasma treated media or STAT3 inhibitor *in vitro,* we treated sphere cultures at day 7 and then we grew them in tumorspheres culture conditions to assay the ability of the drug to inhibit the formation of tumorspheres for 72 h.

### Statistics

For the statistics analysis in the results shown in the figures, 95 % confidence intervals were determined, calculating the mean and standard deviation of the 3 independent experiments. Student T test was used for determining significant differences comparing each treatment with control (untreated). Also, the combination was found to be significant compared to plasma treated medium and to S3I-201 treatment alone. One-way ANOVA analysis is indicated in the figure where * means *p < 0.05;* ** means *p < 0.01* and *** means *p < 0.001).*

## Claims

1. A composition comprising:
a. a liquid or a hydrogel comprising reactive oxygen and nitrogen species (RONS); and
b. a STAT3 inhibitor.

2. The composition according to the preceding claim, wherein the RONS comprise between 10 and 3000 µM H₂O₂, preferably between 10 and 600 µM H₂O₂, more preferably between 10 and 300 µM H₂O₂, even more preferably between 20 and 250 µM H₂O₂.

3. The composition according to any one of the preceding claims, wherein the RONS comprise between 10 and 800 µM NO₂⁻, preferably between 10 and 400 µM NO₂⁻, more preferably between 10 and 250 µM NO₂⁻, even more preferably between 20 and 250 µM NO₂⁻.

4. The composition according to any one of the preceding claims, wherein the liquid is an aqueous medium.

5. The composition according to any one of the preceding claims, wherein the hydrogel is an aqueous solution comprising at least one of gelatin, a gelatin derivative such as metacrylated gelatin, fibrin, fibronectin, collagen, a collagen derivative, alginate, agarose, cellulose, modified cellulose such as hydroxypropyl cellulose, carboxymethylcellulose or hydroxyethyl cellulose, xantan gum, polyethyleneglycol, hyaluronic acid, chitosan, polylactide-co-glycolide, polyhydroxyalcanoates.

6. The composition according to any one of the preceding claims, wherein the hydrogel is an aqueous solution comprising gelatin, alginate, collagen or mixtures thereof.

7. The composition according to any one of the preceding claims, further comprising a ceramic material comprising calcium.

8. The composition according to the preceding claim, wherein the ceramic material comprising calcium is selected from calcium phosphate, hydroxyapatite, calcium deficient hydroxyapatite, brushite, fluorapatite, calcium- sodium and potassium-phosphate, calcium- and sodium- phosphate, calcium- and potassium- phosphate, calcium pyrophosphate, calcium carbonate, calcium sulphate, calcium sulphate hemihydrate, calcium oxide, calcium hydroxide, and mixtures thereof, preferably the ceramic material is hydroxyapatite, brushite, tricalcium phosphate or mixtures thereof.

9. The composition according to any one of the preceding claims, wherein the STAT3 inhibitor prevents Stat3 expression, STAT3 phosphorylation, STAT3 dimerization, STAT3 translocation to the nucleus, STAT3 DNA binding or STAT3 mediated transcription.

10. The composition according to any one of the preceding claims, wherein the STAT3 inhibitor prevents STAT3 phosphorylation, more preferably the STAT3 inhibitor prevents STAT3 phosphorylation at tyrosine 705.

11. The composition according to any one of the preceding claims, wherein the STAT3 inhibitor is S3I-201 or BBI608.

12. The composition according to any one of the preceding claims, further comprising at least an active agent selected from a chemotherapeutic agent and an immunotherapeutic agent.

13. The composition according to any one of the preceding claims for use in the treatment of cancer.

14. The composition for use according to the preceding claim, wherein the cancer is selected from bone cancer, prostate cancer, breast cancer, brain cancer, or colon cancer.

15. The composition for use according to the preceding claim, wherein the bone cancer is osteosarcoma.
